# EUROPEAN PATENT APPLICATION

(11) **EP 1 895 013 A1**
(43) Date of publication of application: **05.03.2008**
(21) Application number: 06017904.1
(22) Date of filing: 28.08.2006
(51) Int. Cl.: C12Q 1/68

(54) **Sensitive method for detecting circular telomeric molecules and its application in cancer diagnostics and treatment**

(71) Applicant: Gregor Mendel Institute of Molecular Plant Biology, 1030 Wien (AT); Austria Wirtschaftsservice Gesellschaft mit beschränkter Haftung, 1030 Wien (AT)
(72) Inventor: Riha, Karel, 1220 Wien (AT)
(74) Representative: Krauss, Jan

(57) **Abstract**

The present invention relates to a method for diagnosing rapid telomere deletion (RTD) in cancer cells, comprising the steps of a) providing a cancer sample containing DNA to be analyzed, b) removing linear DNA molecules from said sample, c) telomere-specific rolling-circle amplification of circular fragments in said sample, and d) detection of amplified telomere-specific molecules in said sample, wherein the presence and/or amount of said telomere-specific molecules is an indication for an RTD in said cancer cells. The present invention furthermore relates to other related uses of said method and kits for cancer diagnosis and therapy.

SEQUENCE LISTING

<110> Gregor Mendel Institute
      Austria Wirtschaftsservice Gesellschaft mbH

<120> Sensitive method for detecting circular telomeric molecules and
      its application in cancer diagnostics and treatment

<130> FB17620

<160> 8

<170> PatentIn version 3.3

<210> 1
<211> 21
<212> DNA
<213> Arabidopsis thaliana

<400> 1
tttagggttt agggtttagg g                                                21


<210> 2
<211> 21
<212> DNA
<213> Arabidopsis thaliana

<400> 2
ccctaaaccc taaaccctaa a                                                21


<210> 3
<211> 21
<212> DNA
<213> Homo sapiens

<400> 3
ttagggttag ggttagggtt a                                                21


<210> 4
<211> 21
<212> DNA
<213> Homo sapiens

<400> 4
ccctaaccct aaccctaacc c                                                21


<210> 5
<211> 14
<212> DNA
<213> Arabidopsis thaliana

<400> 5
ccctaaaccc taaa                                                        14


<210> 6
<211> 14
<212> DNA
<213> Arabidopsis thaliana

<400> 6
tttagggttt aggg                                                        14


<210> 7
<211> 18

<212>  DNA
<213>  Homo sapiens

<400>  7
ccctaaccct aaccctaa                                                    18


<210>  8
<211>  18
<212>  DNA
<213>  Homo sapiens

<400>  8
tttaggttta ggtttagg                                                    18

## Description

The present invention relates to a method for diagnosing rapid telomere deletion (RTD) in cancer cells, comprising the steps of a) providing a cancer sample containing DNA to be analyzed, b) removing linear DNA molecules from said sample, c) telomere-specific rolling-circle amplification of circular fragments in said sample, and d) detection of amplified telomere-specific molecules in said sample, wherein the presence and/or amount of said telomere-specific molecules is an indication for an RTD in said cancer cells. The present invention furthermore relates to other related uses of said method and kits for cancer diagnosis and therapy.

### Field of the invention

### Telomeres and cancer

Telomeres are nucleoprotein structures that protect chromosome ends from being recognized and processed as DNA double strand breaks. Loss of telomere function elicits DNA damage response which can lead to cell senescence or apoptosis. Telomeres are synthesized and maintained by a unique reverse transcriptase, called telomerase. In the absence of the enzyme, telomeres gradually shorten due to the end replication problem. If telomerase activity is not reestablished, continuously dividing cells ultimately lose telomeric sequence critical for chromosome end protection. Hence, telomerase is essential for indefinite cell proliferation.

In humans, telomerase is suppressed in majority of somatic cells with the exception of germ-line and stem cells. As consequence, telomere length in many different cell types inversely correlates with age and telomere erosion is proposed to act as a "biological clock" restricting cell proliferation capacity (Bekaert S, Derradji H, Baatout S. 2004. Telomere biology in mammalian germ cells and during development. Dev Biol 274: 15-30, Lansdorp PM. 2005. Major cutbacks at chromosome ends. Trends Biochem Sci 30: 388-95, Ulaner GA. 2004. Telomere maintenance in clinical medicine. Am J Med 117: 262-9). Telomerase is reactivated in more than 90% of tumors and a high level is required for sustained proliferative activity of cancer cells. Therefore, telomerase is very attractive target for antitumor therapy. Lack or only a very low level of telomerase in somatic cells predicts a high selectivity of telomerase-based therapies for tumor cells and low toxicity. Several therapeutic strategies based on telomeres and telomerase are under development. These include telomerase inhibitors, telomerase promoter-driven gene therapy and telomerase immunotherapy (Shay JW, Wright WE. 2006. Telomerase therapeutics for cancer: challenges and new directions. Nat Rev Drug Discov, Ulaner GA. 2004. Telomere maintenance in clinical medicine. Am J Med 117: 262-9).

Telomerase inhibitors are predicted to induce telomere shortening and chromosome ends deprotection followed by senescence or apoptosis of rapidly proliferating tumor cells. Several classes of chemicals targeting either core components of telomerase complex or telomeres itself have been examined in their ability to suppress proliferation of tumor cells (Odago FO, Gerson SL. 2003. Telomerase inhibition and telomere erosion: a two pronged strategy in cancer therapy. Trends Pharmacol Sci 24: 328-31, Saretzki G. 2003. Telomerase inhibition as cancer therapy. Cancer Lett 194: 209-19). For example, the antisense agent GRN163L, developed by Geron Corporation, has already entered phase II clinical trials for treatment patients with chronic lymphocytic leukemia (www.geron.com).

Although telomerase inhibitors can efficiently suppress proliferation of cancer cells, the major caveat of this approach is that in most cases antiproliferative effects become apparent only after a relatively long lag phase. This is caused by slow attrition of telomeric DNA in the absence telomerase (see section below). Therefore, long treatment times in order of 100 days are necessary to efficiently reduced telomere length (by several kb) even in tumor cultures grown in vitro. These observations led to the suggestion that the use of telomerase inhibitors may be limited only to tumors with relatively short telomeres or for treatments in combination with other conventional therapies to prevent cancer recurrences (Shay JW, Wright WE. 2002. Telomerase: a target for cancer therapeutics. Cancer Cell 2: 257-65).

### Rapid telomere deletion

Telomeres can be shortened by a homologous recombination-based mechanism called rapid telomere deletion (RTD). RTD was first time described in budding yeast, where size of over-extended telomeres was reduced in a single step to a wild type length (Bucholc M, Park Y, Lustig AJ. 2001. Intrachromatid excision of telomeric DNA as a mechanism for telomere size control in Saccharomyces cerevisiae. Mol Cell BioI 21: 6559-73, Li B, Lustig AJ. 1996. A novel mechanism for telomere size control in Saccharomyces cerevisiae. Genes Dev 10: 1310-26). The RTD is presumably mediated by intrachromatid recombination: a single stranded 3' end of the telomere invades internal tracks of double stranded telomeric region forming a displacement loop, which is followed by branch migration and Holliday junction resolution. Products of the recombination reaction are an extrachromsomal telomeric circle and a shortened telomere. RTD-derived deletions can be cytologically detected on metaphase chromosomes by fluorescence *in situ* hybridization.

It is known that the frequency of RTD can be analyzed by scoring sister chromatids with uneven telomeric signal after fluorescence *in situ* hybridization on metaphase chromosomes (Wang RC, Smogorzewska A, de Lange T. 2004. Homologous recombination generates T-loop-sized deletions at human telomeres. Cell 119: 355-68). The limitation of the technique is that it requires preparation of mitotic chromosomes for FISH.

Level of RTD can also be determined by detecting circular telomeric molecules. Telomeric circles ("t-circles") are relatively stable molecules that can persist in cells for several divisions. Two methods have been used for detecting telomeric circles: two-dimensional gel electrophoresis (Larrive M, Wellinger RJ. 2006. Telomerase- and capping-independent yeast survivors with alternate telomere states. Nat Cell Biol) (Wang RC, Smogorzewska A, de Lange T. 2004. Homologous recombination generates T-loop-sized deletions at human telomeres. Cell 119: 355-68) and electron microscopy (Cesare AJ, Griffith JD. 2004. Telomeric DNA in ALT cells is characterized by free telomeric circles and heterogeneous t-loops. Mol Cell Biol 24: 9948-57). However, both approaches are a rather laborious and require high amount of DNA.

### Rolling circle amplification

The principle of rolling circle amplification is copied from bacterial plasmid replication. Several publications in the mid-1990s described molecular technique for *in vitro* rolling nucleic acid synthesis from small singlestranded DNA minicircles using highly processive DNA polymerase. This technique is also called rolling circle amplification (RCA). The major advantages of RCA are linear mode of amplification, high sensitivity, robustness and possibility to use high-fidelity polymerases. For example, phi-29 polymerase can synthesize up to several hundreds of kilobases of ssDNA from small circular templates. Due to these features, RCA methodology led to a development of many applications in nucleic acid and protein detection, called RCA diagnostics. The applications include detection of point mutations using single template molecules (Lizardi PM, Huang X, Zhu Z, Bray-Ward P, Thomas DC, Ward DC. 1998. Mutation detection and single-molecule counting using isothermal rolling-circle amplification. Nat Genet 19: 225-32), diagnostics of viruses with circular genomes (Rector A, Tachezy R, Van Ranst M. 2004. A sequence-independent strategy for detection and cloning of circular DNA virus genomes by using multiply primed rolling-circle amplification. J Virol 78: 4993-8) or cell free cloning (Hutchison CA, 3rd, Smith HO, Pfannkoch C, Venter JC. 2005. Cell-free cloning using phi29 DNA polymerase. Proc Natl Acad Sci US A 102: 17332-6). A comprehensive list of applications is provided in the review of Demidov (Demidov VV. 2002. Rolling-circle amplification in DNA diagnostics: the power of simplicity. Expert Rev Mol Diagn 2: 542-8).

In view of the above, it is the object of this invention to develop of a new and highly sensitive technique for detecting circular telomeric molecules in biological samples in order to improve cancer diagnosis and treatment. Other objects of the present invention will become apparent upon the description as provided herein.

This object of the present invention, in a first aspect thereof, is solved by a method for diagnosing rapid telomere deletion (RTD) in cancer cells. The method comprises the steps of a) providing a cancer sample containing DNA to be analyzed, b) removing linear DNA molecules from said sample, c) telomere-specific rolling-circle amplification of circular fragments in said sample, and d) detection of amplified telomere-specific molecules in said sample, wherein the presence and/or amount of said telomere-specific molecules is an indication for an RTD in said cancer cells.

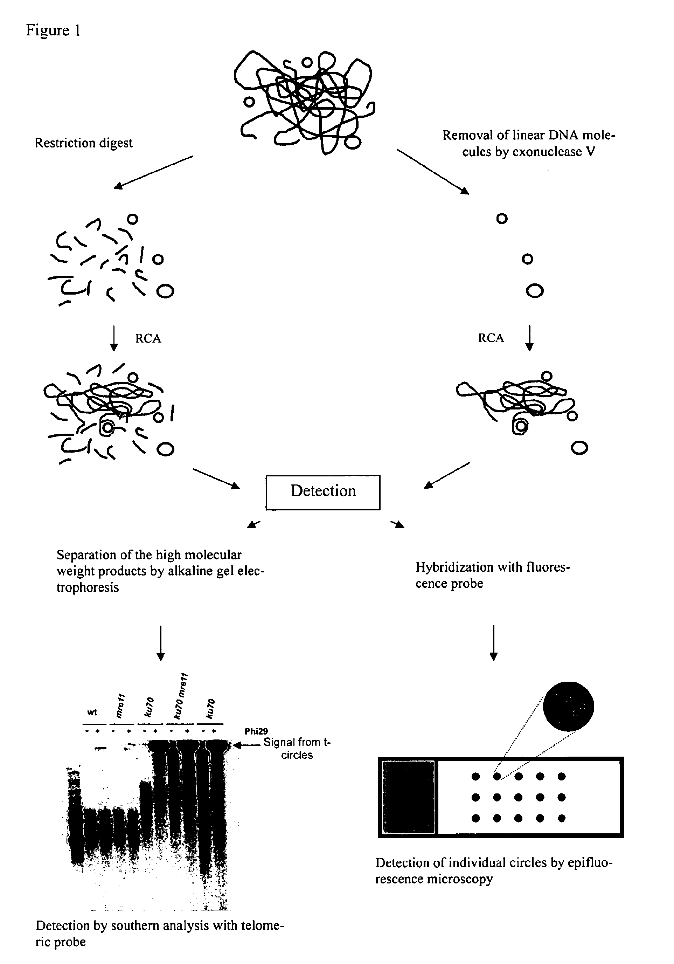

The present invention generally has two parts: first, a general technique for sensitive detection of the t-circles is provided (here abbreviated as TCA; telomeric circle amplification), and, second, this detection of t-circles in human cells is used as a diagnostic marker, preferably for predicting success of telomerase-based anti-tumor therapy and/or for developing RTD-related novel therapeutic approaches. The inventive technique uses rolling circle amplification and it is schematically outlined in preferred embodiments in Figure 1.

The inventors discovered that inactivation of an evolutionary conserved Ku complex in Arabidopsis results in a high frequency of RTD (unpublished data). However, telomeres in Ku-deficient plants are functional and ku mutants do not exhibit any developmental defects. These observations indicate that telomerase effectively counteracts RTD events in Ku-deficient cells. However, a concomitant inactivation of telomerase gene in Ku-null plants results in accelerated loss of telomeric sequence and progressive onset of developmental defects (Riha K, Shippen DE. 2003. Ku is required for telomeric C-rich strand maintenance but not for end-to-end chromosome fusions in Arabidopsis. Proc Natl Acad Sci U S A 100: 611-5). These results indicated that RTD together with telomerase dysfunction represents a lethal combination that can result in a rapid telomere dysfunction and cessation of cell proliferation.

Although RTD is obviously suppressed in somatic cells, data obtained in human HeLa cells (Wang RC, Smogorzewska A, de Lange T. 2004. Homologous recombination generates T-loop-sized deletions at human telomeres. Cell 119: 355-68) and the work of the inventors in *Arabidopsis* suggest that the suppression is not complete. Because majority of cancer cells express telomerase that can efficiently counteract RTD, there is a less stringent pressure to keep the level or RTD low. Therefore, the inventors expected the existence of tumor cell lines that exhibit a high level of RTD. Furthermore, such RTD positive tumors appear to be very sensitive to telomerase inhibition.

Preferred is a method for diagnosing rapid telomere deletion (RTD) in cancer cells according to the present invention, wherein said cancer sample is selected from cancerous tissue, biopsy material, cancer cell lines, tumor tissue, metastatic tissue, and removed organs. In principle, any DNA-containing sample can be used, as long as it potentially contains t-circles. Further preferred is a method for diagnosing rapid telomere deletion (RTD) in cancer cells according to the present invention, further comprising a preparation and/or extraction of the DNA from said sample. Respective methods for extraction of DNAs are well known in the state of the art. Generally, the method must be suitable for the preparation and/or extraction of DNA containing t-circles. The cancer cells can be derived from cancerous diseases, such as Neuroblastoma, cervical carcinoma, Adrenal Cancer; Anal Cancer; Bladder Cancer; Brain Tumors; Breast Cancer; Cervical Cancer; Chronic Lymphocytic Leukemia (CLL); Chronic Myelogenous Leukemia (CML); Colorectal Cancer; Endometrial and Uterine Cancer; Esophageal Cancer; Ewing's Sarcoma; Fallopian Tube Cancer; Gallbladder Cancer; Gastric Cancer; Hairy Cell Leukemia; Kaposi's Sarcoma; Kidney Cancer; Laryngeal Cancer; Leukemias, such as Acute Lymphocytic Leukemia (ALL) or Acute Myelogenous Leukemia (AML); Li-Fraumeni Syndrome; Liver Cancer; Lung Cancer; Lymphomas; Medulloblastoma; Melanoma; Mesothelioma; Myelomas; Non-Small Cell Lung Cancer; Oropharyngeal Cancers; Ovarian Cancer; Pancreatic Cancer; Parathyroid Cancer; Penile Cancer; Pituitary Cancer; Prostate Cancer; Retinoblastoma; Rhabdomyosarcoma and Other Soft-Tissue Sarcomas; Osteosarcoma; Small Intestine Cancers; Small-Cell Lung Cancer; Testicular Cancer; Thymoma; Thyroid Cancer; Urethral Cancer; Vaginal Cancer; Vulvar Cancer, and Wilms' Tumor.

In a next step of the method for diagnosing rapid telomere deletion (RTD) in cancer cells according to the present invention, linear DNA molecules are removed from said sample. Preferably, removing of said linear DNA molecules from said sample is selected from digestion with a frequently cutting restriction enzyme and/or treatment with exonuclease V. This step represents one of the major technical challenges, since it had to be assured that the long extension products are indeed primed from t-circles and not from telomeric sequence located on chromosomes. In one preferred embodiment, the frequent cutters (i.e. restriction enzymes having a short recognition sequence of, e.g., 4 bases in length), which can be used prior to RCA reaction are AluI, MseI, HhaI or Tru9I, whereas the t-circles are insensitive to the digest. Chromosomal DNA is thus digested to small linear t-fragments, which do not provide a template of sufficient length to prime synthesis of high molecular weight products. Low molecular products derived from the chromosome can be easily distinguished from the high molecular weight products primed from the t-circles, e.g., by alkaline agarose electrophoresis. Further methods for distinguishing the circles as high molecular weight products from the small chromosomal fragments are known to the person of skill and described in the respective literature.

The inventors further improved this technique by employing exonuclease V, which specifically degrades linear DNA molecules, but not circles. The combination of the restriction enzyme and exonuclease V treatments are important steps of the protocol that essentially completely eliminate linear DNA molecules and leave only intact t-circles. The exonuclease step thus significantly improves the specificity and sensitivity of the inventive technique.

An alternative embodiment of the method for diagnosing rapid telomere deletion (RTD) in cancer cells according to the present invention, then further comprises a preparation and/or extraction of the circular DNA from said sample. This step can be employed in order to provide essentially pure t-circles. Methods for purifying the circles as high molecular weight products from the small chromosomal fragments are known to the person of skill and described in the respective literature.

In a next step of the method for diagnosing rapid telomere deletion (RTD) in cancer cells according to the present invention, a telomere-specific rolling-circle amplification of circular fragments in said sample (rolling-circle amplification, RCA) is performed. Said rolling-circle amplification comprises telomere-specific primers and a suitable polymerase.

Preferred is a method for diagnosing rapid telomere deletion (RTD) in cancer cells according to the present invention, wherein said telomere-specific primers are selected from primers having the sequences ^{5'}TTTAGGGTTTAGGGTTTAGGG^{3'} (SEQ ID No. 1) or ^{5'}CCCTAAACCCTAAACCCTAAA^{3'} (SEQ ID No. 2) for plant-derived primers, or ^{5'}TTAGGGTTAGGGTTAGGGTTA^{3'} (SEQ ID No. 3) or ^{5'}CCCTAACCCTAACCCTAACCC^{3'} (SEQ ID No. 4) for human primers. Preferably, the primers may contain a modification at the 3'-end in order to prevent nuclease activity of the polymerase, more preferably a phosphorotioate modification of the last three 3' nucleotides to improve resistance against weak intrinsic 3' to 5' nuclease activity of the phi-29 polymerase. Furthermore, a suitable polymerase is used, such as phi29-polymerase, Vent DNA Polymerase or T7 Sequenase. The amplification can be further enhanced by using Thermus thermophilus SSB mutant protein (Inoue J, Shigemori Y, Mikawa T. Improvements of rolling circle amplification (RCA) efficiency and accuracy using Thermus thermophilus SSB mutant protein. Nucleic Acids Res. 2006 May 17;34(9):e69).

There is a plenty of examples about using RCA in many biotechnology applications. In one example, small synthetic telomeric circles are used as a template for synthesis of artificial telomeric sequences (Lindstrom UM, Chandrasekaran RA, Orbai L, Helquist SA, Miller GP, ct al. 2002. Artificial human telomeres from DNA nanocircle templates. Proc Natl Acad Sci US A 99: 15953-8). However, to the knowledge of the present inventors, so far nobody has used RCA based technique for detecting t-circles in tumor diagnostics.

Preferably, the rolling-circle amplification according to the present inventions produces single stranded telomeric molecules having a length of more than about 100 kb, in order to allow an efficient detection thereof. Preferred is a method for diagnosing rapid telomere deletion (RTD) in cancer cells according to the present invention, wherein said rolling-circle amplification can be repeated using TCA product as a template for another round of polymerization.

In a next step of the method for diagnosing rapid telomere deletion (RTD) in cancer cells according to the present invention, the amplified telomere-specific molecules in said sample are detected. Preferred is a method for diagnosing rapid telomere deletion (RTD) in cancer cells according to the present invention, wherein said detection of said telomeric molecules comprises Southern hybridization and epifluorescence microscopy. Labeling of the probes as used is preferably done with a fluorescent label, nevertheless, also other labels can be used, such as radioactive labels, enzymatic labels, etc.

To further improve sensitivity of the assay, the inventors detected individual TCA products by epifluorescence microscopy using a modified protocol according to Lizardi et al. (Lizardi PM, Huang X, Zhu Z, Bray-Ward P, Thomas DC, Ward DC. 1998. Mutation detection and single-molecule counting using isothermal rolling-circle amplification. Nat Genet 19: 225-32). The inventors thus hybridized fluorescently labeled oligonucleotide probe(s) complementary to a telomeric sequence ^{5'}CCCTAAACCCTAAA^{3'} (SEQ ID No. 5) or ⁵'TTTAGGGTTTAGGG^{3'} (SEQ ID No. 6) derived from plants or ^{5'}CCCTAACCCTAACCCTAA^{3'} (SEQ ID No. 7) or ^{5'}TTTAGGTTTAGGTTTAGG^{3'} (SEQ ID No. 8) derived from human with products of the TCA reaction in a test tube. Preferably (more preferably Cy3 or FITC conjugated) peptide nucleic acid (PNA) probes are used, nevertheless, (more preferably Cy3 or FITC conjugated) deoxyribonucleotide probes can also be used

The inventors then spotted different dilutions of the reaction on slides and examined them under epifluorescence microscope. Individual TCA products in dilutions containing less than 5 ng of total DNA could be detected. Analysis of telomeric circles by microscopy is much faster than gel electrophoresis and Southern hybridization and does not require work with radioactivity. Thus, detection of the telomeric circles by TCA followed by fluorescence microscopy is extremely efficient and can further be optimized for a routine use in clinical laboratories. Thus, in another aspect thereof, the present invention can be performed in an automated environment, such as robots which are controlled by computers running a suitable software, preferably for both controlling the processes and interpretation of the results as obtained.

Figure 1 summarizes preferred embodiments of the method according to the present invention. DNA extracted from a biological sample is either digested by a frequently cutting restriction enzyme (left path) or treated with exonuclease V, which specifically removes linear DNA molecules (right path) or treated with both a restricition enzyme and exonuclease V. Genomic DNA is indicated by black lines, telomeric sequences are marked in gray. Because t-circles (indicated in dark gray) are resistant to such treatments, these steps increase the specificity for RCA. The t-circles then serve as template for RCA using telomere-specific primers and phi-29-polymerase. This leads to a synthesis of more than 100 kb long single stranded telomeric molecules. The molecules can preferably be detected either by Southern hybridization (left path) or by labeling with a fluorescence probe and epifluorescence microscopy (right path).

The autoradiogram in bottom left corner shows detection of the t-circles in Arabidopsis mutants deficient for the Ku70 gene, showing the sensitivity of the method. Product of the TCA (indicated by arrow) can not efficiently enter gel and remain in loading wells. Using this approach it is possible to detect only weak signals coming from t-circles in wild type cells. The typical amount of the total DNA used for the experiment is 500 ng, and sensitivity can be, alternatively) enhanced by application of further amplification steps using TCA product as a template for another round of polymerization (branched-RCA). We also succeeded with detecting individual t-circles using the fluorescence hybridization approach indicated on the left. Total amount of the DNA needed for this approach may be scaled down to the orders of nanograms.

In summary, this invention provides a new and highly sensitive technique for detecting circular telomeric molecules in biological samples. Experiments made by the inventors in *Arabidopsis* demonstrate a high sensitivity of the RCA-based method for the detection of t-circles, Although there is great evolutionary distance between plants and animals, telomere structure and function is extremely conserved. Human telomeric sequence differs from the *Arabidopsis* sequence only by one nucleotide (TTAGGG vs. TTTAGGG), therefore this technique is almost directly applicable to human samples.

Another preferred aspect of the method for diagnosing rapid telomere deletion (RTD) in cancer cells according to the present invention further comprises a step of identifying the stage, phase, type, treatment response and/or relapse of cancer, based, at least in part, on said RTD in said cancer cells. As an example, the level and/or frequency of RTD can be compared between cancer and non-cancer cells, and differences can be attributed to, for example, the metastasis in said cancer (when compared to non-metastatic cells), or a tumor relapse (if the level of RTD changes after a certain period of time).

Telomerase inhibitors provide a new promising strategy for cancer treatment, and several drugs have already entered clinical trials. However, as discussed above, their long-term application may be required to suppress telomere function and effectively repress tumor growth. Furthermore, telomerase inhibitors are predicted not to be very efficient against tumors with relatively long telomeres. Thus, telomerase inhibitors will have profound effect only in a subset of tumors. It will be very beneficial for clinical diagnosis to identify patients which are susceptible to this type of drugs prior to treatment. Targeted application of telomerase inhibitors would minimize risk of potential side effects and reduce cost of the treatment. Tumors with a high level of RTDs will be particularly sensitive to telomerase inhibitors.

TCA is a relatively simple method which enables fast identification of the cells exhibiting RTD, and to determine the level and/or frequency thereof. Therefore, the invention comprises the use of TCA as a diagnostic technique for predicting success of cancer therapy based on telomerase inhibitors. Furthermore, the invention provides the cellular components of RTD as a novel therapeutic approach for cancer therapy, where the RTD is increased in a manner that the cells will undergo senescence and cell death.

Yet another preferred aspect of the method for diagnosing rapid telomere deletion (RTD) in cancer cells according to the present invention thus further identifies cancer cells that are sensitive or insensitive against telomerase inhibition and/or an increase of RTD, based, at least in part, on said RTD in said cancer cells. Those cells can be identified by a high level of RTD, when compared to non-cancer cells or less sensitive cancer cells, where the telomerase activity is comparably lower. As a general rule, the higher the RTD-level in a cell, the more the cell is assumed to be sensitive to telomerase inhibition. Based on these results, in still another preferred aspect of the method for diagnosing rapid telomere deletion (RTD) in cancer cells according to the present invention said method further comprises predicting the success of telomerase-based anti-tumor therapy based on said sensitivity or insensitivity, based, at least in part, on said RTD in said cancer cells. Again, as a general rule, the higher the RTD-level in a cell, the more the cell is assumed to be sensitive to telomerase inhibition.

Thus, the ability to efficiently assess level of RTD in cancer cells is an important diagnostic tool for predicting success of telomerase-based anti-tumor therapy.

Another preferred aspect then relates to a method for screening for anti-cancer agents, comprising performing a method according to the present invention both in the presence and absence of a potential anti-cancer agent, and identifying an agent that increases the amount of RTD in said cancer cell.

The method of screening according to the present invention can be performed in several different formats. One embodiment is a method, wherein the assay is performed *in vitro.* The screening assays of the present invention preferably involve the use of cancer cell-lines and other cells, as long as these cells exhibit RTD.

An additional embodiment of the present invention relates to a method wherein the assay is performed *in vivo.* Preferably, the assay is performed in a mouse or rat. In general, the *in vivo* assay will not be substantially different from the above-mentioned in vitro assay. In a general screening assay for anti-cancer agents will be provided in that the agent to be tested is/are administered to a mouse or a rat. Then, it will be determined, if said agent leads to an increase or decrease of RTD, compared to the absence of the agent to be tested, wherein a difference identifies a potential anti-cancer agent. Of course, these assays can be performed in other non-human mammals as well. In a second step, the anti-cancer activity of the competitive ligand itself can be tested, e.g. in an assay for the effect on the level of RTD or the expression of proteins of the RTD-machinery in the cell. Accordingly, preferred is a method according to the present invention, wherein said potential anti-cancer agent increases the level of RTD in said cancer cell.

An additional embodiment of the present invention relates to a method according to the invention, wherein said agent is selected from a library of naturally occurring or synthetic compounds which are randomly tested for the influence on RTD. Such libraries and the methods how to build up such a library, as well as methods for using these libraries for the screening of candidate agents are well known to the person skilled in the art and further described in the respective literature. Furthermore, some of these libraries are commercially available. The present invention contemplates high throughput screening of agents that have an effect on RTD. The agents as described above, and modifications of said agents, including analogues, derivatives, fragments, active moieties, and the like, may be screened using methods and systems of the present invention.

Potential agents include small organic molecules, peptides, polypeptides and antibodies. For example, a compound that promotes RTD may be a small molecule that binds to and, preferably, occupies proteins of the RTD cellular machinery (mechanism), such as the TRF2, Pot1 and Ku proteins telomere capping proteins, thereby causing a shift in the RTD. Examples of small molecules include, but are not limited to, small organic molecule, peptides or peptide-like molecules. Other potential agents include RTD-machinery genes-antisense molecules.

Another preferred embodiment of the present invention relates to a method for the production of a pharmaceutical formulation, comprising a screening method as above, and formulating the agent as identified with suitable auxiliary agents and/or pharmaceutical carriers. Such formulations therefore include, in addition to the agent, a physiologically acceptable carrier or diluent, possibly in admixture with one or more other agents such as other agents or drugs, such as another anti-cancer agent. Suitable carriers include, but are not limited to, physiological saline, phosphate buffered saline, phosphate buffered saline glucose and buffered saline. Alternatively the agent may be lyophilized (freeze dried) and reconstituted for use when needed by the addition of an aqueous buffered solution as described above. Routes of administration are routinely parenteral, including intravenous, intramuscular, subcutaneous and intraperitoneal injection or delivery. The administration can be systemic and/or locally.

Preferred pharmaceutical compositions according to the present invention comprise at least one agent as identified according to the present invention, and a pharmaceutically acceptable carrier as above. A preferred pharmaceutical composition according to the present invention comprise at least one agent as identified according to the present invention, together with a telomerase inhibiting substance (telomerase inhibitor). Examples for telomerase inhibitors are DODC (diethyloxadicarbocyanine), Lovaxin T (Advaxis Inc.), quinoline-based inhibitors, such as G4t-459 and G4T-405 (Aventis), BIBR-1532 (Boehringer Ingelheim), fluoroindolo-carbazoles (FICZ; Bristol-Myers Squibb), RHPS-4 (Cancer Research Technology Ltd.), 6-formyl-pyridine-2-carboxylate-derivatives (Chong Kun Dang Pharmaceutical Corp.), antisense molecules, UTAG-1 and -2 (Dana-Farber Cancer Inst.), GRN-163L (Geron Corp.), 3,6-disubstituted acridine derivatives (e.g. BRACO-19; Inst Cancer Res, UK), telomerase inhibitors containing a bisindole unit (e.g. Mitsubishi Pharma Corp.), Telometastin (WO 00/24747; Tokyo University), 2,6-disubstituted anthraquinones (e.g. BSU-1051; University of Texas System), and tetra-(N-methyl-4-pyridyl)porphine (University of Texas System).

The pharmaceutical composition can be used for topical or parenteral administration, such as subcutaneous, intradermal, intraperitoneal, intravenous, intramuscular or oral administration. For this, the agents are dissolved or suspended in a pharmaceutically acceptable, preferably aqueous carrier. In addition, the composition can contain excipients, such as buffers, binding agents, blasting agents, diluents, flavors, lubricants, etc. The composition can be used for a prevention, prophylaxis and/or therapy of cancerous diseases that are related to an increased RTD. The pharmaceutical preparation of the present invention, containing at least one of the agents of the present invention, is administered to a patient that suffers from a cancerous disease, in particular is selected from Neuroblastoma, cervical carcinoma, Adrenal Cancer; Anal Cancer; Bladder Cancer; Brain Tumors; Breast Cancer; Cervical Cancer; Chronic Lymphocytic Leukemia (CLL); Chronic Myelogenous Leukemia (CML); Colorectal Cancer; Endometrial and Uterine Cancer; Esophageal Cancer; Ewing's Sarcoma; Fallopian Tube Cancer; Gallbladder Cancer; Gastric Cancer; Hairy Cell Leukemia; Kaposi's Sarcoma; Kidney Cancer; Laryngeal Cancer; Leukemias, such as Acute Lymphocytic Leukemia (ALL) or Acute Myelogenous Leukemia (AML); Li-Fraumeni Syndrome; Liver Cancer; Lung Cancer; Lymphomas; Medulloblastoma; Melanoma; Mesothelioma; Myelomas; Non-Small Cell Lung Cancer; Oropharyngeal Cancers; Ovarian Cancer; Pancreatic Cancer; Parathyroid Cancer; Penile Cancer; Pituitary Cancer; Prostate Cancer; Retinoblastoma; Rhabdomyosarcoma and Other Soft-Tissue Sarcomas; Osteosarcoma; Small Intestine Cancers; Small-Cell Lung Cancer; Testicular Cancer; Thymoma; Thyroid Cancer; Urethral Cancer; Vaginal Cancer; Vulvar Cancer, and Wilms' Tumor.

Another aspect of the present invention relates to the agent as identified according to the present invention or the pharmaceutical composition according to the present invention for use in medicine. Preferred is a use of the pharmaceutical composition according to the present invention as an anti-cancer agent.

Another aspect of the present invention then relates to a method for diagnosing a cancerous disease, comprising a method according to the present invention as above, and identifying said cancerous disease based, at least in part, on said RTD in said cell. A distortion of the RTD in said cell compared to a normal non-cancerous cell is an indication for a cancerous disease. In addition, also the frequency and the level of the RTD in said cell to be diagnosed can be used to characterize said disease, preferably in order to distinguish between different cancer types.

Yet another preferred aspect of the present invention then relates to a method for an improved treatment of a cancerous disease, comprising administering to a patient in need of said treatment an effective amount of the pharmaceutical composition as produced according to the present invention as above. Preferred is a method for an improved treatment of a cancerous disease according to the present invention, wherein said cancerous disease is selected from Neuroblastoma, cervical carcinoma, Adrenal Cancer; Anal Cancer; Bladder Cancer; Brain Tumors; Breast Cancer; Cervical Cancer; Chronic Lymphocytic Leukemia (CLL); Chronic Myelogenous Leukemia (CML); Colorectal Cancer; Endometrial and Uterine Cancer; Esophageal Cancer; Ewing's Sarcoma; Fallopian Tube Cancer; Gallbladder Cancer; Gastric Cancer; Hairy Cell Leukemia; Kaposi's Sarcoma; Kidney Cancer; Laryngeal Cancer; Leukemias, such as Acute Lymphocytic Leukemia (ALL) or Acute Myelogenous Leukemia (AML); Li-Fraumeni Syndrome; Liver Cancer; Lung Cancer; Lymphomas; Medulloblastoma; Melanoma; Mesothelioma; Myelomas; Non-Small Cell Lung Cancer; Oropharyngeal Cancers; Ovarian Cancer; Pancreatic Cancer; Parathyroid Cancer; Penile Cancer; Pituitary Cancer; Prostate Cancer; Retinoblastoma; Rhabdomyosarcoma and Other Soft-Tissue Sarcomas; Osteosarcoma; Small Intestine Cancers; Small-Cell Lung Cancer; Testicular Cancer; Thymoma; Thyroid Cancer; Urethral Cancer; Vaginal Cancer; Vulvar Cancer, and Wilms' Tumor. Suitable formulations, routes of administrations and dosages are indicated above. Another aspect of the present invention relates to the use of an agent according to the present invention or the pharmaceutical composition according to the present invention for the manufacture of a medicament for the prevention and/or treatment of a cancerous disease, wherein said cancerous disease is selected from Neuroblastoma, cervical carcinoma, Adrenal Cancer; Anal Cancer; Bladder Cancer; Brain Tumors; Breast Cancer; Cervical Cancer; Chronic Lymphocytic Leukemia (CLL); Chronic Myelogenous Leukemia (CML); Colorectal Cancer; Endometrial and Uterine Cancer; Esophageal Cancer; Ewing's Sarcoma; Fallopian Tube Cancer; Gallbladder Cancer; Gastric Cancer; Hairy Cell Leukemia; Kaposi's Sarcoma; Kidney Cancer; Laryngeal Cancer; Leukemias, such as Acute Lymphocytic Leukemia (ALL) or Acute Myelogenous Leukemia (AML); Li-Fraumeni Syndrome; Liver Cancer; Lung Cancer; Lymphomas; Medulloblastoma; Melanoma; Mesothelioma; Myelomas; Non-Small Cell Lung Cancer; Oropharyngeal Cancers; Ovarian Cancer; Pancreatic Cancer; Parathyroid Cancer; Penile Cancer; Pituitary Cancer; Prostate Cancer; Retinoblastoma; Rhabdomyosarcoma and Other Soft-Tissue Sarcomas; Osteosarcoma; Small Intestine Cancers; Small-Cell Lung Cancer; Testicular Cancer; Thymoma; Thyroid Cancer; Urethral Cancer; Vaginal Cancer; Vulvar Cancer, and Wilms' Tumor. Suitable formulations, routes of administrations and dosages are indicated above.

The present invention further provides a diagnostic kit for detecting and/or diagnosing RTD, comprising materials for measuring RTD according to the methods according to the present invention, optionally together with manuals, chemicals and buffers. The kit can also be used for diagnosing and/or monitoring cancer according to a method of the present invention, as described above.

The present invention shall now be further described in the following examples with reference to without being limited thereto. For the purposes of the present invention, all references as cited herein are incorporated by reference in their entireties. In the Sequences and Figures,

SEQ ID No. 1 to SEQ ID No. 4 show primers as used in the context of the present invention.

SEQ ID No. 5 to SEQ ID No. 8 show probes as used in the context of the present invention.
Figure 1 shows two preferred embodiments of the method according to the present invention. DNA extracted from a biological sample is either digested by a frequently cutting restriction enzyme (left path) or treated with exonuclease III, which specifically removes linear DNA molecules (right path). Genomic DNA is indicated by black lines, telomeric sequences are marked in gray. Because t-circles (indicated in dark gray) are resistant to such treatments, these steps increase specificity RCA. The t-circles then serve as template for RCA using telomere-specific primers and phi-29 polymerase. This leads to a synthesis of more than 100 kb long single stranded telomeric molecules. The molecules can be detected either by Southern hybridization (left path) or by labeling with fluorescence probe and epifluorescence microscopy (right path). The autoradiogram in bottom left corner shows detection of the t-circles in *Arabidopsis* mutants deficient for the *Ku70* gene. Product of the TCA indicated by red arrow can not efficiently enter gel and remain in loading wells. Using this approach we can also detect weak signal coming from t-circles in wild type cells. The typical amount of the total DNA used for the experiment is 500 ng, and sensitivity can be enhanced by application of further amplification steps using TCA product as a template for another round of polymerization (branched-RCA). The inventors also succeeded with detecting individual t-circles using the fluorescence hybridization approach indicated on the left. Total amount of the DNA needed for this approach may be scaled down to the orders of ngs.

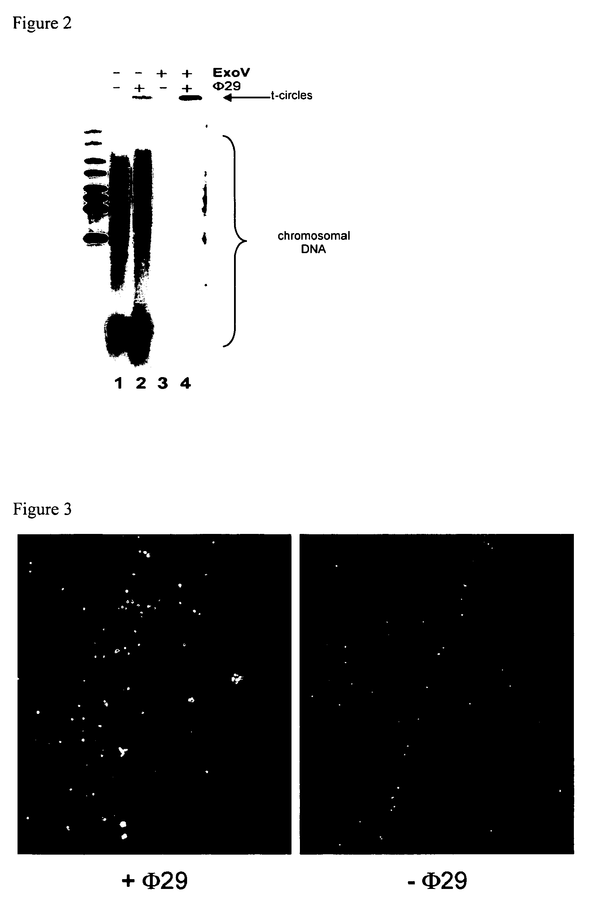

Figure 2 shows that exonuclease V specifically eliminates signal from non-circular DNA molecules, and increases intensity of the signal from t-circles.
Figure 3 shows the detection of the TCA products by epifluorescence microscopy. Individual products of TCA are apparent as bright dots on the left picture. The picture on the right shows "background" fluorescence from a control sample without phi-29 polymerase. 5 ng of total genomic DNA subjected to the TCA reaction was spotted on a slide. Magnification 1000x.

### Example

This example provides a brief lineout of the experiment as schematically depicted in Figure 1, with exemplary results depicted in Figure 2 and 3.
1. The DNA is extracted from human sample (using standard technique).
2. 0.5 µg to 10 µg of the DNA is digested with frequently cutting restriction endonuclease (MseI, AluI, Tru9I etc) in an appropriate restriction buffer.
3. This step is alternative to the step 2: DNA is dissolved in the exonuclease V buffer (66.7 mM glycine-NaOH buffer pH9.4, 30 mM MgCl2, 8.3 mM 2-mercaptoethanol, 0.5 mM ATP) and incubated with 6 U of exonuclease V at 37°C for 10 min.
4. Circular DNA is precipitated with ethanol in the presence of carrier RNA or glycogen or PEG.
5. Circular DNA is resuspended in primer annealing buffer (0.2 M Tris pH7.5, 0,2 M KCI, 1 mM EDTA) in the presence of either 1 µM ^{5'}TTAGGGTTAGGGTTAGGGTTA^{3'} or ^{5'}CCCTAACCCTAACCCTAACCC^{3'} deoxyribonucleotides with phosphorotioate modification.
6. Circular DNA is heat denatured (5 min at 96°C) and reaction is cooled to the 25°C to allow primer annealing.
7. Rolling circle amplification is performed in Phi-29 buffer (33 mM Tris-Acetate pH7.9; 10 mM magnesium acetate, 66 mM potassium acetate, 0.1% Tween 20, 1 mM DTT, 0.375 mM dNTPs and 7.5 U of Phi-29 polymerase) at 30°C for 16 hrs.
8. Phi29 polymerase is heat inactivated and products of extension are either analyzed by alkaline gel electrophoresis and Southern hybridization with radioactively labelled telomeric probe or by hybridization with fluorescently labelled PNA probe and epifluorescent microscopy.

## Claims

1. A method for diagnosing rapid telomere deletion (RTD) in cancer cells, comprising
a) providing a cancer sample containing DNA to be analyzed,
b) removing linear DNA molecules from said sample,
c) telomere-specific rolling-circle amplification of circular fragments in said sample, and
d) detection of amplified telomere-specific molecules in said sample,
wherein the presence and/or amount of said telomere-specific molecules is an indication for an RTD in said cancer cells.

2. The method for diagnosing rapid telomere deletion (RTD) in cancer cells according to claim 1, wherein said cancer sample is selected from cancerous tissue, biopsy material, cancer cell lines, tumor tissue, metastatic tissue, and removed organs.

3. The method for diagnosing rapid telomere deletion (RTD) in cancer cells according to claim 1 or 2, further comprising a preparation and/or extraction of the DNA from said sample.

4. The method for diagnosing rapid telomere deletion (RTD) in cancer cells according to any of claims 1 to 3, wherein removing said linear DNA molecules from said sample is selected from digestion with a frequently cutting restriction enzyme and/or treatment with exonuclease V.

5. The method for diagnosing rapid telomere deletion (RTD) in cancer cells according to claim 4, further comprising a preparation and/or extraction of the circular DNA from said sample.

6. The method for diagnosing rapid telomere deletion (RTD) in cancer cells according to any of claims 1 to 5, wherein said rolling-circle amplification comprises telomere-specific primers and a suitable polymerase.

7. The method for diagnosing rapid telomere deletion (RTD) in cancer cells according to claim 6, wherein said polymerase is selected from phi29-polymerase, Vent DNA Polymerase, and T7 Sequenase.

8. The method for diagnosing rapid telomere deletion (RTD) in cancer cells according to any of claims 1 to 7, wherein said rolling-circle amplification produces single stranded telomeric molecules having a length of more than about 100 kb.

9. The method for diagnosing rapid telomere deletion (RTD) in cancer cells according to any of claims 1 to 8, wherein said rolling-circle amplification can be repeated using the product as a template for another round of polymerization.

10. The method for diagnosing rapid telomere deletion (RTD) in cancer cells according to any of claims 1 to 9, wherein said detection of said telomeric molecules comprises Southern hybridization and/or epifluorescence microscopy.

11. The method for diagnosing rapid telomere deletion (RTD) in cancer cells according to any of claims 1 to 10, wherein said method further identifies the stage, phase, type, treatment response and/or relapse of cancer, based, at least in part, on said RTD in said cancer cells.

12. The method for diagnosing rapid telomere deletion (RTD) in cancer cells according to any of claims 1 to 10, wherein said method further identifies cancer cells that are sensitive or insensitive against telomerase inhibition and/or the promotion of RTD, based, at least in part, on said RTD in said cancer cells.

13. The method for diagnosing rapid telomere deletion (RTD) in cancer cells according to claim 10, wherein said method further comprises predicting the success of telomerase-based and/or promotion of RTD-based anti-tumor therapy based on said sensitivity or insensitivity, based, at least in part, on said RTD in said cancer cells.

14. A method for diagnosing a cancerous disease, comprising a method according to any of claims 1 to 13, and identifying said cancerous disease based on said method.

15. A method for monitoring the success of treatment of a cancerous disease, comprising performing the method according to any of claims 1 to 13 after a treatment of said cancer, wherein a change in the RTD as detected is used, at least in part, to monitor the success of treatment of the cancerous disease.

16. A method for screening for anti-cancer agents, comprising performing a method according to any of claims 1 to 13 in the presence and absence of a potential anti-cancer agent, and identifying an agent that increases the amount of RTD in said cancer cell.

17. The method according to claim 16, wherein said potential anti-cancer agent promotes RTD in said cancer cell.

18. A method for producing a pharmaceutical composition, comprising a method according to claim 16 or 17, and formulating the agent as identified with suitable auxiliary agents and/or pharmaceutical carriers, optionally together with a telomerase inhibitor.

19. A method for an improved treatment of a cancerous disease, comprising administering to a patient in need of said treatment an effective amount of the pharmaceutical composition as produced according to claim 18.

20. A method for an improved treatment of a cancerous disease according to claim 19, wherein said cancerous disease is selected from Neuroblastoma, cervical carcinoma, Adrenal Cancer; Anal Cancer; Bladder Cancer; Brain Tumors; Breast Cancer; Cervical Cancer; Chronic Lymphocytic Leukemia (CLL); Chronic Myelogenous Leukemia (CML); Colorectal Cancer; Endometrial and Uterine Cancer; Esophageal Cancer; Ewing's Sarcoma; Fallopian Tube Cancer; Gallbladder Cancer; Gastric Cancer; Hairy Cell Leukemia; Kaposi's Sarcoma; Kidney Cancer; Laryngeal Cancer; Leukemias, such as Acute Lymphocytic Leukemia (ALL) or Acute Myelogenous Leukemia (AML); Li-Fraumeni Syndrome; Liver Cancer; Lung Cancer; Lymphomas; Medulloblastoma; Melanoma; Mesothelioma; Myelomas; Non-Small Cell Lung Cancer; Oropharyngeal Cancers; Ovarian Cancer; Pancreatic Cancer; Parathyroid Cancer; Penile Cancer; Pituitary Cancer; Prostate Cancer; Retinoblastoma; Rhabdomyosarcoma and Other Soft-Tissue Sarcomas; Osteosarcoma; Small Intestine Cancers; Small-Cell Lung Cancer; Testicular Cancer; Thymoma; Thyroid Cancer; Urethral Cancer; Vaginal Cancer; Vulvar Cancer, and Wilms' Tumor.
